# EUROPEAN PATENT APPLICATION

(11) **EP 1 391 509 A1**
(43) Date of publication of application: **25.02.2004**
(21) Application number: 01938587.1
(22) Date of filing: 11.06.2001
(51) Int. Cl.: C12N 15/09, A01K 67/04, C12N 9/02

(54) **TRANSFORMED SILKWORM PRODUCING HUMAN COLLAGEN**

(30) Priority: 18.04.2001 JP 2001120155
(71) Applicant: JAPAN SCIENCE AND TECHNOLOGY CORPORATION, Kawaguchi-shi, Saitama 332-0012 (JP); Hiroshima Industrial Promotion Organization, Hiroshima-shi, Hiroshima 730-0052 (JP); TERUMO KABUSHIKI KAISHA, Tokyo 151-0072 (JP); KOKEN CO., LTD., Toshima-ku, Tokyo 171-0031 (JP); National Institute of Agrobiological Sciences, Tsukuba-shi, Ibaraki 305-8602 (JP)
(72) Inventor: YOSHIZATO, Katsutoshi, Higashihiroshima-shi, Hiroshima 739-0144 (JP); TOMITA, Masahiro, Higashihiroshima-shi, Hiroshima 739-0025 (JP); SATOU, Tsutomu, Kita-ku, Tokyo (JP); MORI, Hajime, Kyoto-shi, Kyoto 603-8131 (JP); TAMURA, Toshiki, Tsukuba-shi, Ibaraki 305-0851 (JP); ADACHI, Takahiro, Higashihiroshima-shi, Hiroshima 739-0044 (JP); MUNETSUNA, Hiroto, Kamo-gun, Hiroshima (JP)
(74) Representative: Webber, Philip Michael
(86) International application number: PCT/JP2001/004906
(87) International publication number: WO 2002/086119

(57) **Abstract**

The present invention provides a transformed silkworm which has a polynucleotide encoding human collagen within the genomic DNA and produces recombinant human collagen as a part of proteins in the cocoon or the silk gland; a process for producing recombinant human collagen by using this transformed silkworm; and a recombinant vector for use in the generation of the transformed silkworm. Since human collagen is collected from the cocoon discharged by this transformed silkworm or its silk gland, highly pure human collagen can be conveniently obtained in a large amount. Moreover, because the recombinant human collagen produced by the transformed silkworm is safe collagen which is free from any fear of the contamination with pathogens such as viruses or prions and exhibits no antigenicity toward humans. Thus, it can be utilized in various industrial fields including medicines, foods, cosmetics and the like.

SEQUENCE LISTING

<110> Japan Science and Technology Corporation;
      Hiroshima Industrial Technology Organization;
      TERUMO CORPORATION;
      TOKEN Co., Ltd.; and
      National Institute of Agrobiological Sciences

<120> A Transformed Silkworm producing Human Collagen

<130> 01-F-027PCT

<140> PCT/JP01/04906
<141> 2001-06-11

<150> JP2000-361563
<151> 2000-11-28

<160> 29

<170> PatentIn Ver. 2.1

<210> 1
<211> 2483
<212> DNA
<213> Bombyx mori

<220>
<221> CDS
<222> (228)..(1880)

<400> 1
tacgcggggg aaccgaccga cgtcgggctg cgtggacgca cgtatctttg ccagattgtc 60

gctttattta acgtttataa ttgtgaattg attgcaattc aatttgtggt gaatgaggtt 120

aaactgcaat acgtttttct aataatcctt aatgatgtac gtttgtctgt aaaacaattt 180

gctgatctgc aagttctaga atcgttttca tatttatttc tgcaaaa atg gaa aca 236
                                                    Met Glu Thr
                                                     1

gta aga gcg ata gct ttt ttg ctt ctg ttt ttc ttt act tgg gcc aaa 284

Val Arg Ala Ile Ala Phe Leu Leu Leu Phe Phe Phe Thr Trp Ala Lys
    5                   10                  15

gca gag cta ttc aca gcc ata acg gat gtt gaa ccg cta ctg gaa acc 332
Ala Glu Leu Phe Thr Ala Ile Thr Asp Val Glu Pro Leu Leu Glu Thr
20                  25                  30                  35

cac aag agg atc ata gac gat tta gat gat tac ctg caa aaa gaa gag 380
His Lys Arg Ile Ile Asp Asp Leu Asp Asp Tyr Leu Gln Lys Glu Glu
                40                  45                  50

agg aga ctt ttc act ttg aag aaa cac ttg aat tta tat aaa agg gaa 428
Arg Arg Leu Phe Thr Leu Lys Lys His Leu Asn Leu Tyr Lys Arg Glu
            55                  60                  65

cac gaa agg gct atg gat gac atc cct aac tac ctc ggc aac ccg atc 476
His Glu Arg Ala Met Asp Asp Ile Pro Asn Tyr Leu Gly Asn Pro Ile
        70                  75                  80

aat gct ttc acg ttg ata aaa aga tta acg gcc gac ctt gat ttt ata 524
Asn Ala Phe Thr Leu Ile Lys Arg Leu Thr Ala Asp Leu Asp Phe Ile
    85                  90                  95

gag gat agc att aaa att gga aca gaa tac ata aag aac gtc aca atg 572
Glu Asp Ser Ile Lys Ile Gly Thr Glu Tyr Ile Lys Asn Val Thr Met
100                 105                 110                 115

aac cac gtg gac gtg aaa tat ccg tca ttg gaa gat ctg acg gga gca 620
Asn His Val Asp Val Lys Tyr Pro Ser Leu Glu Asp Leu Thr Gly Ala
                120                 125                 130

gct cag gcg ctg act cgg ttg cag gaa acc tac tat tta aat gta cac 668
Ala Gln Ala Leu Thr Arg Leu Gln Glu Thr Tyr Tyr Leu Asn Val His
            135                 140                 145

gat cta gcc gag ggt ata ttg aat gga gtt tca tac agt act cct atg 716
Asp Leu Ala Glu Gly Ile Leu Asn Gly Val Ser Tyr Ser Thr Pro Met
        150                 155                 160

acg gcg agc gat tgt tac gaa ctc ggc cgg act cta tac aac gat aaa 764
Thr Ala Ser Asp Cys Tyr Glu Leu Gly Arg Thr Leu Tyr Asn Asp Lys
    165                 170                 175

gat tac aca aac gcc ttg gcc tgg atg aaa gaa gcg cta agg aaa tat 812
Asp Tyr Thr Asn Ala Leu Ala Trp Met Lys Glu Ala Leu Arg Lys Tyr
180                 185                 190                 195

aaa gat gaa aac gtc atg tac ccg ttc acc gaa gtc gat atc ttg gaa 860
Lys Asp Glu Asn Val Met Tyr Pro Phe Thr Glu Val Asp Ile Leu Glu
                200                 205                 210

tat ata gga ttc gca tat tat tta aac gga gac gtc aaa acc gct ctg 908
Tyr Ile Gly Phe Ala Tyr Tyr Leu Asn Gly Asp Val Lys Thr Ala Leu
            215                 220                 225

gaa tgg act cag aga ctt ctg tcc gtc gat ccg aag cac gta cga gcg 956
Glu Trp Thr Gln Arg Leu Leu Ser Val Asp Pro Lys His Val Arg Ala
        230                 235                 240

cgg ggc aac ata ccg cac tat cag aag acg ata gcc gaa caa gag gcc 1004
Arg Gly Asn Ile Pro His Tyr Gln Lys Thr Ile Ala Glu Gln Glu Ala
    245                 250                 255

gaa tta aag aag caa caa cgt ggg gaa acc tcg gat gag cct gaa gaa 1052
Glu Leu Lys Lys Gln Gln Arg Gly Glu Thr Ser Asp Glu Pro Glu Glu
260                 265                 270                 275

gaa gat ggt caa gat tac gaa tta tca gag tac gca aag gaa cgc aaa 1100
Glu Asp Gly Gln Asp Tyr Glu Leu Ser Glu Tyr Ala Lys Glu Arg Lys
                280                 285                 290

gtt tac gaa tcg ctg tgt cgg gga gaa atg gaa ata ccc cat gag att 1148
Val Tyr Glu Ser Leu Cys Arg Gly Glu Met Glu Ile Pro His Glu Ile
            295                 300                 305

act aag agg ttg aaa tgt tgg tac gtc acc gac acg cat ccg ttt tta 1196
Thr Lys Arg Leu Lys Cys Trp Tyr Val Thr Asp Thr His Pro Phe Leu
        310                 315                 320

aag ttg gcc cca atc aaa gtg gag cag atg tac gtg aag ccc gac ata 1244
Lys Leu Ala Pro Ile Lys Val Glu Gln Met Tyr Val Lys Pro Asp Ile
    325                 330                 335

ttt atg ttc cac gaa gtg atg acc gac gac gag att gag ttc atc aaa 1292
Phe Met Phe His Glu Val Met Thr Asp Asp Glu Ile Glu Phe Ile Lys
340                 345                 350                 355

aaa cga gca aag ccg agg ttc aaa cgg gct gtc gtt cac gac cct aaa 1340
Lys Arg Ala Lys Pro Arg Phe Lys Arg Ala Val Val His Asp Pro Lys
                360                 365                 370

act ggt gag ctg aca ccg gcc cat tac cgc atc agc aag tcg tcg tgg 1388
Thr Gly Glu Leu Thr Pro Ala His Tyr Arg Ile Ser Lys Ser Ser Trp
            375                 380                 385

ctc cgc gac gag gag tct ccg gtc ata gcg cgc atc acg cag cgc gtc 1436
Leu Arg Asp Glu Glu Ser Pro Val Ile Ala Arg Ile Thr Gln Arg Val
        390                 395                 400

acc gac atg acc ggg ctc agc atg ctg cac gcc gag gag ctt cag gtc 1484
Thr Asp Met Thr Gly Leu Ser Met Leu His Ala Glu Glu Leu Gln Val
    405                 410                 415

gtc aac tac ggc ata ggg gga cac tac gaa ccg cac ttc gac ttc gct 1532
Val Asn Tyr Gly Ile Gly Gly His Tyr Glu Pro His Phe Asp Phe Ala
420                 425                 430                 435

agg aaa cgt gag aat cca ttc acg aaa ttc ggc ggc aac aga ata gcc 1580
Arg Lys Arg Glu Asn Pro Phe Thr Lys Phe Gly Gly Asn Arg Ile Ala
                440                 445                 450

acc gtc ctc ttc tac atg tct gac gtg gcg cag ggc ggc gct aca gtg 1628
Thr Val Leu Phe Tyr Met Ser Asp Val Ala Gln Gly Gly Ala Thr Val
            455                 460                 465

ttc acc gaa ctt gga ctc agt ttg ttt cca ata aaa cga gct gcg gcg 1676
Phe Thr Glu Leu Gly Leu Ser Leu Phe Pro Ile Lys Arg Ala Ala Ala
        470                 475                 480

ttc tgg ttg aac ctg cac gcg tcg ggc gaa gga gac ctc gcc acc agg 1724
Phe Trp Leu Asn Leu His Ala Ser Gly Glu Gly Asp Leu Ala Thr Arg
    485                 490                 495

cat gcc gcc tgc ccc gtg ctc agg gga tcc aag tgg gtg tca aat aaa 1772
His Ala Ala Cys Pro Val Leu Arg Gly Ser Lys Trp Val Ser Asn Lys
500                 505                 510                 515

tgg ata cat caa ggc ggg caa gag ctg ttg agg ccc tgc gac ctt gag 1820
Trp Ile His Gln Gly Gly Gln Glu Leu Leu Arg Pro Cys Asp Leu Glu

520                    525                    530

tac cag gag gag ggc atc atc cgc aag att cct cgt cca gtg ccg aag    1868
Tyr Gln Glu Glu Gly Ile Ile Arg Lys Ile Pro Arg Pro Val Pro Lys
            535                    540                    545

aca tcc agg tag actccgagca acgtgtgacc gaacggaact gatcataaat    1920
Thr Ser Arg
        550

tgttcggtat agtgcatttt ttttttaaat aaaatagatt aaatattaca tagtataata 1980

gaatgttggg tctgtgtagt acacgtattc gacttctgtg tctcgtgcga ttttacgaat 2040

aaccacaagc ataatcgact agaatgcata atttttaatt agttatgggt caagaaagaa 2100

gattctcatt atgaaagacg ccaagaaaat tacgccaact aacttttagg agatgtttta 2160

aataaccaat aatttgagtg caatttatac tatactttta atatatttat taaattgttc 2220

caatttcttt actttcccct atttagaacg tatagtttaa gttaacgttg atttgtttga 2280

ttgtaaaata ttaacagtaa actcacacca ggcacacaca aaaaaccatc tattgttgcg 2340

aacaaattca caaataaaca tatctaataa aaaaaaatgt taaataataa ataagttgca 2400

atgaaacact aatatatttt aacatattat atattatagt gatatcttag aataaaccat 2460

cgtggctcga tggctcgtgg cta                                          2483

<210> 2
<211> 550
<212> PRT
<213> Bombyx mori

<400> 2
Met Glu Thr Val Arg Ala Ile Ala Phe Leu Leu Leu Phe Phe Phe Thr
1               5                   10                  15
Trp Ala Lys Ala Glu Leu Phe Thr Ala Ile Thr Asp Val Glu Pro Leu
            20                  25                  30
Leu Glu Thr His Lys Arg Ile Ile Asp Asp Leu Asp Asp Tyr Leu Gln
        35                  40                  45

Lys Glu Glu Arg Arg Leu Phe Thr Leu Lys Lys His Leu Asn Leu Tyr
    50                  55                  60
Lys Arg Glu His Glu Arg Ala Met Asp Asp Ile Pro Asn Tyr Leu Gly
65                  70                  75                  80
Asn Pro Ile Asn Ala Phe Thr Leu Ile Lys Arg Leu Thr Ala Asp Leu
                85                  90                  95
Asp Phe Ile Glu Asp Ser Ile Lys Ile Gly Thr Glu Tyr Ile Lys Asn
            100                 105                 110
Val Thr Met Asn His Val Asp Val Lys Tyr Pro Ser Leu Glu Asp Leu
        115                 120                 125
Thr Gly Ala Ala Gln Ala Leu Thr Arg Leu Gln Glu Thr Tyr Tyr Leu
    130                 135                 140
Asn Val His Asp Leu Ala Glu Gly Ile Leu Asn Gly Val Ser Tyr Ser
145                 150                 155                 160
Thr Pro Met Thr Ala Ser Asp Cys Tyr Glu Leu Gly Arg Thr Leu Tyr
                165                 170                 175
Asn Asp Lys Asp Tyr Thr Asn Ala Leu Ala Trp Met Lys Glu Ala Leu
            180                 185                 190
Arg Lys Tyr Lys Asp Glu Asn Val Met Tyr Pro Phe Thr Glu Val Asp
        195                 200                 205
Ile Leu Glu Tyr Ile Gly Phe Ala Tyr Tyr Leu Asn Gly Asp Val Lys
    210                 215                 220
Thr Ala Leu Glu Trp Thr Gln Arg Leu Leu Ser Val Asp Pro Lys His
225                 230                 235                 240
Val Arg Ala Arg Gly Asn Ile Pro His Tyr Gln Lys Thr Ile Ala Glu
                245                 250                 255
Gln Glu Ala Glu Leu Lys Lys Gln Gln Arg Gly Glu Thr Ser Asp Glu
            260                 265                 270
Pro Glu Glu Glu Asp Gly Gln Asp Tyr Glu Leu Ser Glu Tyr Ala Lys
        275                 280                 285
Glu Arg Lys Val Tyr Glu Ser Leu Cys Arg Gly Glu Met Glu Ile Pro
    290                 295                 300
His Glu Ile Thr Lys Arg Leu Lys Cys Trp Tyr Val Thr Asp Thr His
305                 310                 315                 320
Pro Phe Leu Lys Leu Ala Pro Ile Lys Val Glu Gln Met Tyr Val Lys
                325                 330                 335
Pro Asp Ile Phe Met Phe His Glu Val Met Thr Asp Asp Glu Ile Glu
            340                 345                 350
Phe Ile Lys Lys Arg Ala Lys Pro Arg Phe Lys Arg Ala Val Val His
        355                 360                 365
Asp Pro Lys Thr Gly Glu Leu Thr Pro Ala His Tyr Arg Ile Ser Lys
    370                 375                 380
Ser Ser Trp Leu Arg Asp Glu Glu Ser Pro Val Ile Ala Arg Ile Thr

385                 390                 395                 400
Gln Arg Val Thr Asp Met Thr Gly Leu Ser Met Leu His Ala Glu Glu
                405                 410                 415
Leu Gln Val Val Asn Tyr Gly Ile Gly Gly His Tyr Glu Pro His Phe
            420                 425                 430
Asp Phe Ala Arg Lys Arg Glu Asn Pro Phe Thr Lys Phe Gly Gly Asn
        435                 440                 445
Arg Ile Ala Thr Val Leu Phe Tyr Met Ser Asp Val Ala Gln Gly Gly
    450                 455                 460
Ala Thr Val Phe Thr Glu Leu Gly Leu Ser Leu Phe Pro Ile Lys Arg
465                 470                 475                 480
Ala Ala Ala Phe Trp Leu Asn Leu His Ala Ser Gly Glu Gly Asp Leu
                485                 490                 495
Ala Thr Arg His Ala Ala Cys Pro Val Leu Arg Gly Ser Lys Trp Val
            500                 505                 510
Ser Asn Lys Trp Ile His Gln Gly Gly Gln Glu Leu Leu Arg Pro Cys
        515                 520                 525
Asp Leu Glu Tyr Gln Glu Glu Gly Ile Ile Arg Lys Ile Pro Arg Pro
    530                 535                 540
Val Pro Lys Thr Ser Arg
545                 550

<210> 3
<211> 22
<212> DNA
<213> Artificial sequence

<220>
<213> Description of Artificial Sequence: Synthesized oligonucleotide

<400> 3
tcgtaactgc ctacacgttt gc                                          22

<210> 4
<211> 20
<212> DNA
<213> Artificial sequence

<220>
<213> Description of Artificial Sequence: Synthesized oligonucleotide

<400> 4
agacgtgaac ctggctggct                                                    20

<210> 5
<211> 15
<212> DNA
<213> Artificial sequence

<220>
<213> Description of Artificial Sequence: Synthesized oligonucleotide

<400> 5
tttagactcg agcct                                                         15

<210> 6
<211> 25
<212> DNA
<213> Artificial sequence

<220>
<213> Description of Artificial Sequence: Synthesized oligonucleotide

<400> 6
tacagttctt atctagacgt gaacc                                              25

<210> 7
<211> 33
<212> DNA
<213> Artificial sequence

<220>
<213> Description of Artificial Sequence: Synthesized oligonucleotide

<400> 7
gacataatat gtgacgctcg agaattagac tgc                                     33

<210> 8
<211> 22
<212> DNA

<213> Artificial sequence

<220>
<213> Description of Artificial Sequence: Synthesized oligonucleotide

<400> 8
ggatccagac atgataagat ac                                                22


<210> 9
<211> 21
<212> DNA
<213> Artificial sequence

<220>
<213> Description of Artificial Sequence: Synthesized oligonucleotide

<400> 9
gatcataatc agccatacca c                                                 21


<210> 10
<211> 22
<212> DNA
<213> Artificial sequence

<220>
<213> Description of Artificial Sequence: Synthesized oligonucleotide

<400> 10
gatcccccta gaatcccaaa ac                                                22

<210> 11
<211> 22
<212> DNA
<213> Artificial sequence

<220>
<213> Description of Artificial Sequence: Synthesized oligonucleotide

<400> 11
cttagcgacg tgttcacttt gc                                                22

<210> 12
<211> 27
<212> DNA
<213> Artificial sequence

<220>
<213> Description of Artificial Sequence: Synthesized oligonucleotide

<400> 12
agatctagtc gaatttcgac tctgcg                                          27


<210> 13
<211> 21
<212> DNA
<213> Artificial sequence

<220>
<213> Description of Artificial Sequence: Synthesized oligonucleotide

<400> 13
cccgatgata agacgactat g                                               21


<210> 14
<211> 21
<212> DNA
<213> Artificial sequence

<220>
<213> Description of Artificial Sequence: Synthesized oligonucleotide

<400> 14
ccaggttcac gtctaaataa g                                               21


<210> 15
<211> 27
<212> DNA
<213> Artificial sequence

<220>

<213> Description of Artificial Sequence: Synthesized oligonucleotide

<400> 15
agatctcatg acaacagtac cgaaatc                                    27


<210> 16
<211> 21
<212> DNA
<213> Artificial sequence

<220>
<213> Description of Artificial Sequence: Synthesized oligonucleotide

<400> 16
taacagacca ctaaaatgaa g                                          21


<210> 17
<211> 27
<212> DNA
<213> Artificial sequence

<220>
<213> Description of Artificial Sequence: Synthesized oligonucleotide

<400> 17
ctcgagtagc ttttccatca tcaatgt                                    27


<210> 18
<211> 33
<212> DNA
<213> Artificial sequence

<220>
<213> Description of Artificial Sequence: Synthesized oligonucleotide

<400> 18
tctagaagat ctggtacggt tcgtaaagtt cac                             33


<210> 19

<211> 21
<212> DNA
<213> Artificial sequence

<220>
<213> Description of Artificial Sequence: Synthesized oligonucleotide

<400> 19
gtctgttatg tgaccaatcg g                                                21


<210> 20
<211> 21
<212> DNA
<213> Artificial sequence

<220>
<213> Description of Artificial Sequence: Synthesized oligonucleotide

<400> 20
acagaccact aaaatgaagc c                                                21


<210> 21
<211> 27
<212> DNA
<213> Artificial sequence

<220>
<213> Description of Artificial Sequence: Synthesized oligonucleotide

<400> 21
ctcgagcctg gctggctgct tgtgcaa                                          27


<210> 22
<211> 21
<212> DNA
<213> Artificial sequence
<220>
<213> Description of Artificial Sequence: Synthesized oligonucleotide

<400> 22

tgttccacgg aaacactggt g                                                21

<210> 23
<211> 21
<212> DNA
<213> Artificial sequence

<220>
<213> Description of Artificial Sequence: Synthesized oligonucleotide

<400> 23
catcctccag aactgtgtag g                                                21

<210> 24
<211> 22
<212> DNA
<213> Artificial sequence

<220>
<213> Description of Artificial Sequence: Synthesized oligonucleotide

<400> 24
cacaacgtct atatcatggc cg                                               22

<210> 25
<211> 21
<212> DNA
<213> Artificial sequence

<220>
<213> Description of Artificial Sequence: Synthesized oligonucleotide

<400> 25
atgttgtggc ggatcttgaa g                                                21

<210> 26
<211> 19
<212> DNA
<213> Artificial sequence

<220>
<213> Description of Artificial Sequence: Synthesized oligonucleotide

<400> 26
tncargtngc naaytaygg                                                    19


<210> 27
<211> 19
<212> DNA
<213> Artificial sequence

<220>
<213> Description of Artificial Sequence: Synthesized oligonucleotide

<400> 27
cnccnccngc nswnacrtc                                                    19


<210> 28
<211> 25
<212> DNA
<213> Artificial sequence

<220>
<213> Description of Artificial Sequence: Synthesized oligonucleotide

<400> 28
gaggacggtg gctattctgt tgccg                                             25


<210> 29
<211> 25
<212> DNA
<213> Artificial sequence

<220>
<213> Description of Artificial Sequence: Synthesized oligonucleotide

<400> 29
ggcatagggg gacactacga accgc                                             25

## Description

### Technical Field

The invention of the present application relates to a transformed silkworm which produces recombinant human collagen. More particularly, the present invention relates to a transformed silkworm which produces recombinant human collagen; a recombinant vector for use in the generation of this transformed silkworm; and a process for producing recombinant human collagen.

### Background Art

Collagen is a major protein which constitutes extracellular matrices, and has various physiological functions controlling cell proliferation, differentiation, migration and the like in addition to mechanical functions to maintain the structure of a living organism by serving as a scaffold of cells. Therefore, collagen has been widely utilized in a medical field as a biomaterial for repairing injury of a living organism (J. Surg. Res. 10: 485-491, 1970), or as a carrier for sustained release of a certain type of drugs (J. Controlled Release 33: 307-315, 1995). However, most collagen which is used at present is derived from an animal tissue such as one from a cattle or a pig. It is known that an allergic response occurs in patients accounting for about 3% when such collagen is transplanted to a human (J. Immunol. 136: 877-882, 1986; Biomaterials 11: 176-180, 1990). Furthermore, dangers of the contamination with pathogens such as viruses or prions in collagen derived from animal tissues have been big problems in recent years. Thus, it has been desired to develop a system for producing recombinant human collagen without antigenicity and dangerous contamination of pathogen. Therefore, the inventors of this application achieved an invention and filed a patent application of a process for producing recombinant human collagen having a triple helical structure which is equivalent to that in a human living body through infecting a recombinant virus having an inserted cDNA encoding human collagen to insect cells (JP-A-8-23979). Moreover, a process for producing human collagen by using mammalian cells or yeast has been also proposed (JP-T-7-501937).

As described above, processes in which insect cells, mammalian cells, or yeast are used have been proposed as the process for producing recombinant human collagen. However, according to the process in which insect cells or mammalian cells are used, to achieve high amount of the production is difficult which allows for utilization in a medical field. Further, according to the process in which yeast is used, the recombinant product is produced in the fungus bodies, therefore, purification of recombinant human collagen is not necessarily easy.

The present invention was accomplished in light of the circumstances as described above, and an object of the invention is to solve the problems in the conventional art and to provide a process for producing recombinant human collagen with high productivity and the easiness in purification as well as the genetic engineering materials for the same.

### Disclosure of Invention

A first aspect of the present invention is a transformed silkworm, which comprises a polynucleotide encoding human collagen within the genomic DNA and produces recombinant human collagen as a part of proteins in the cocoon or the silk gland.

A second aspect of the invention is a transformed silkworm, which comprises a polynucleotide encoding a fusion protein of human collagen with a silkworm silk protein within the genomic DNA and produces the fusion protein as a part of proteins in the cocoon or the silk gland.

Exemplary transformed silkworms according to the first and second aspects of the invention may include adult silkworms, larvae, pupae and eggs.

Further, in a preferred embodiment of the transformed silkworm according to the first and second aspects of the invention, a polynucleotide encoding at least one of the α subunit and the β subunit of prolyl hydroxylase is included within the genomic DNA.

A third aspect of the invention is a process for producing recombinant human collagen, which comprises isolating and purifying recombinant human collagen from the cocoon or the silk gland of the transformed silkworm of the first aspect of the invention.

A fourth aspect of the invention is a process for producing recombinant human collagen, which comprises isolating a fusion protein of recombinant human collagen with a silkworm silk protein from the cocoon or the silk gland of the transformed silkworm of the second aspect, and isolating and purifying recombinant human collagen from the fusion protein.

A fifth aspect of the invention is a fusion protein of recombinant human collagen with a silkworm silk protein produced by the transformed silkworm of the second aspect of the invention.

A sixth aspect of the invention is a targeting vector derived from *Autographa californica* nuclear polyhedrosis virus, which is used for executing homologous recombination of a polynucleotide encoding human collagen to an arbitrary region in a silkworm genome, and producing a transformed silkworm which produces human collagen. In the invention, "polynucleotide encoding human collagen" refers to a nucleic acid molecule which expresses human collagen, and implies a human genomic DNA, an mRNA transcribed from a genomic DNA, a cDNA synthesized from an mRNA or the like.

One embodiment of the targeting vector of the sixth aspect of the invention is that both ends of the polynucleotide encoding human collagen have a DNA sequence homologous to an arbitrary region of a genomic DNA encoding a silkworm silk protein (silkworm silk protein gene). This targeting vector executes homologous recombination of a polynucleotide encoding human collagen downstream of an expression regulatory sequence (promoter/ enhancer sequence) of a silk protein gene of a silkworm genome (i.e., endogenous).

Another embodiment of the targeting vector of this sixth aspect of the invention is that both ends of a human collagen expression cassette have a DNA sequence homologous to an arbitrary region of a silkworm genomic DNA. This targeting vector executes homologous recombination of the human collagen expression cassette to an arbitrary region of a silkworm genome. This "expression cassette" means a fusion polynucleotide including a polynucleotide encoding human collagen ligated under the control of an expression regulatory sequence of a silkworm silk protein gene.

A seventh aspect of this invention is a targeting vector derived from *Autographa californica* nuclear polyhedrosis virus for executing homologous recombination of a polynucleotide encoding at least one of the α subunit and the β subunit of prolyl hydroxylase to an arbitrary region of a silkworm genome.

An eighth aspect of the invention is a recombinant plasmid vector having a human collagen expression cassette in a region sandwiched between a pair of inverted terminal repeats of DNA transposon derived from an insect.

A ninth aspect of the invention is a recombinant plasmid vector having a polynucleotide encoding at least one of the α subunit and the β subunit of prolyl hydroxylase to a region sandwiched between a pair of inverted terminal repeats of DNA transposon derived from an insect.

A tenth aspect of the invention is a set of vectors comprising a recombinant plasmid vector of the aforementioned eighth aspect of the invention, and a recombinant plasmid vector having a polynucleotide encoding transposase of transposon. Through use of this set of vectors, the human collagen expression cassette is transferred to a silkworm genome and thereby a transformed silkworm is generated which produces human collagen.

An eleventh aspect of the invention is a set of vectors comprising a recombinant plasmid vector of the aforementioned ninth aspect of the invention, and a recombinant plasmid vector having a polynucleotide encoding transposase of transposon. Through use of this set of vectors of the eleventh invention and the set of vectors of the aforementioned tenth invention, a transformed silkworm which produces human collagen with hydroxyproline is generated.

A twelfth aspect of the invention is a polynucleotide encoding the α subunit of silkworm prolyl hydroxylase having an amino acid sequence of SEQ ID NO: 2. Specifically, the polynucleotide is a DNA fragment having a base sequence set out in SEQ ID NO: 1.

In a preferred embodiment of each invention of this application, the polynucleotide encoding the prolyl hydroxylase α subunit is at least a coding region of a DNA fragment having the base sequence of SEQ ID NO: 1. Furthermore, in a preferred embodiment, the human collagen expression cassette is a fusion polynucleotide including a polynucleotide encoding human collagen ligated under the control of an expression regulatory sequence of a silkworm silk protein gene, or is a fusion polynucleotide including a polynucleotide encoding a silkworm silk protein and a polynucleotide encoding human collagen ligated under the control of an expression regulatory sequence of a silkworm silk protein gene.

### Brief Description of Drawings

Fig. 1 is a restriction enzyme map of a transfer vector pMOSRA-1.
Fig. 2 is a restriction enzyme map of mini collagen genes incorporated in a *piggyBac* plasmid vector (pMOSRA-4B, pMOSRA-5 and pMOSRA-6).
Fig. 3 is an electrophoresis images of PCR products amplified with genomic DNAs extracted from positive F1 adult silkworms as a template. The number above images, e.g., 1 and 2 show numbers of positive silkworms. The lane M is for a 100 bp ladder marker.
Fig. 4 is an electrophoresis images of RT-PCR products amplified using an RNA extracted from the silk gland of positive F1 larval silkworms of the fifth-instar stage. The electrophoresis was conducted: in the lanes 1 and 2 for RT-PCR products from positive silkworms; and in the lane C for RT-PCR products from a wild type silkworm. The lane M indicates a 100 bp ladder marker.

### Best Mode for Carrying Out the Invention

Collagen has been so far known as existing 19 different types including type I through type XIX. Any of these types of collagen is characteristic in that it is a trimer molecule formed from three subunits (α chain), and has a triple helical structure within its molecule. Among these collagens, some of them are synthesized as follows that procollagen that is a precursor is synthesized at first, and then converted to a mature collagen molecule. For example, fibrous collagen such as type I, II or III is synthesized as procollagen having an amino propeptide and a carboxyl propeptide with non-triple helical structure at an amino terminal end and a carboxyl terminal end of the triple helical region which is a main body thereof, followed by cleavage of both propeptides by specific protease to form mature collagen. In addition, collagen is subjected to a variety of posttranslational modifications including hydroxylation of proline, hydroxylation of lysine, oxidation of lysine and hydroxylysine (modification to aldehyde) and the like during its biosynthetic pathway. In particular, hydroxylation of proline by prolyl hydroxylase is extremely important in achieving stability of collagen at a physiological temperature.

Human collagen which is a subject matter of the invention may be any collagen including type I through type XIX collagens, and also includes any partial amino acid sequence of any one of these collagens. Further, those having partial alteration of the amino acid sequence of these collagens, and those having an added amino acid sequence which is not derived from collagen are also involved. Moreover, in addition to mature collagens, procollagens that are precursors and those having a cleaved part in a propeptide are also included. Additionally, in accordance with the invention, immature collagen molecules such as those with an incomplete posttranslational modification including hydroxylation of proline and those with an incomplete triple helical structure are also included in the subject matter.

Silkworms produce a cocoon through discharging a large amount of a silk thread when entered in the spinning stage. Major components of this silk thread are silk proteins that may include fibroin, P25 and sericin. The amount of synthesis of these silk proteins is enormous which reaches to about 0.5 g in average per one silkworm. The silk proteins are synthesized in an organ which is referred to as a silk gland. The silk gland is constituted from a posterior silk gland, a middle silk gland and an anterior silk gland. Fibroin and P25 are specifically synthesized and secreted in the posterior silk gland, whilst sericin is specifically synthesized and secreted in the middle silk gland, respectively. Fibroin is a complex which is constituted from an H chain and an L chain, and P25 is further associated with this complex (J. Biol. Chem. 275: 40517-40528, 2000). Fibroin and P25 secreted from the posterior silk gland are gradually delivered by a peristaltic movement of the silk gland to the middle silk gland where they are covered circumferentially by sericin secreted therefrom, and then discharged as a silk thread after being further delivered to the anterior silk gland. Hence, the silk gland of a silkworm is an organ having superior ability to synthesize proteins, and thus, when recombinant human collagen is expressed in this organ, eminently high productivity can be expected. Moreover, it is markedly easy to collect and purify the synthesized recombinant human collagen from the cocoon discharged by the silkworm, or from proteins in the silk gland because: the silk thread is excreted from the body; only small kinds of the silk proteins constitute the silk thread; and fibroin which is present in a largest quantity among the silk proteins is insoluble in an aqueous solution.

A transient expression system of a foreign gene in a silkworm is established in which a *Bombyx mori* nuclear polyhedrosis virus (BmNPV) is utilized as a vector (JP-B-7-97995). However, since this process is lethal to a silkworm due to the infection with a virus, the foreign gene can not be transmitted to the following generations. Thus, the expression of a useful protein is limited to one generation. Therefore, viral inoculation must be conducted at every time of allowing expression of the foreign gene. On the other hand, Mori et al. developed a process capable of introducing a gene without killing the silkworm, and transmitting the foreign gene to the next generation via the germ cells in instances of the infection to a female, by way of the infection to a larval silkworm with *Autographa californica* nuclear polyhedrosis virus (AcNPV) (JP-A-6-277051). Further, Yamao et al. succeeded in generating a transformed silkworm having a foreign gene inserted into a fibroin L chain gene of a silkworm genome by gene targeting through the infection of AcNPV having a part of a fibroin L chain gene sequence incorporated therein to a larval silkworm (Genes Dev. 13: 511-516, 1999).

Further, Tamura et al. (Nat. Biotechnol. 18: 81-84, 2000) succeeded in generating a transformed silkworm having an inserted foreign gene by microinjection of a plasmid vector having incorporated *piggyBac* which is DNA transposon derived from a Lepidoptera insect, *Trichoplusia ni*, into a silkworm egg.

In the transformed silkworms generated according to these processes, the inserted foreign gene is maintained within the chromosome without dropout, and persistently expresses a recombinant protein throughout the generations.

In the invention, at first, a polynucleotide encoding human collagen is subjected to incorporation into AcNPV vector or a plasmid vector constructed on the basis of DNA transposon. Subsequently, a transformed silkworm is generated in which a polynucleotide encoding human collagen is incorporated therein genomic sequence by transformation using any one of these vectors.

The polynucleotide encoding human collagen which is capable for use may be a genomic DNA of human collagen, an mRNA or a cDNA synthesized from an mRNA. Preferably, cDNA is used. Although instances in which a cDNA is used are primarily explained below, the polynucleotide used in the invention is not limited to cDNAs.

Human collagen cDNA may be any cDNA of type I through type XIX collagens. Base sequences of these cDNAs are available from information described in a literature (e.g., Essays Biochem. 27: 49-67, 1992; Annu. Rev. Biochem. 64: 403-434, 1995). For example, each cDNA of any one of human type I through type XIX collagens can be obtained by a method in which a human cDNA library is screened using an oligonucleotide produced on the basis of any one of these cDNA sequences as a probe, or a PCR method in which oligonucleotides corresponding to both ends of the cDNA sequence are used as primers and a human DNA is used as a template, or an RT-PCR method in which an RNA extracted from a human cell is used as a template.

In order to allow expression of a human collagen cDNA in a silkworm silk gland cell, expression regulatory sequences (e.g., promoter or enhancer) can be utilized which are derived from silk protein genes that may include a fibroin H chain, a fibroin L chain, P25 and sericin. Through construction of an expression cassette utilizing the promoter and enhancer of any one of these silk protein genes, recombinant human collagen can be expressed in a large amount, in a manner specific to the silk gland. For example, when a promoter and enhancer of the fibroin H chain, the fibroin L chain, P25 are used, expression of human collagen is enabled in a posterior silk gland, whilst when a promoter and enhancer of sericin are utilized, expression of human collagen is enabled in a middle silk gland. The human collagen expression cassette may also permit the synthesis of a fusion protein of human collagen with a silkworm silk protein by producing a fusion polynucleotide of human collagen cDNA with silk protein cDNAs which may include the fibroin H chain, the fibroin L chain, P25 and sericin, and incorporating this fusion polynucleotide into a silkworm genomic sequence in order to facilitate a secretion and silk thread discharged from the silk gland cells. In this instance, the fused protein may be a partial amino acid sequence of a silk protein, or may be a full length amino acid sequence. For example, when a fusion protein having a signal peptide of human collagen substituted for a signal peptide of a silk protein is synthesized, secretion of human collagen from the silk gland cells can be facilitated. In addition, when a fusion protein of human collagen with the full length of fibroin L chain is synthesized for example, thus synthesized fusion protein of human collagen with the fibroin L chain forms a complex with the fibroin H chain via a disulfide bond. Accordingly, more efficient secretion can be effected.

In instances where the introduction system of the gene is a gene targeting method with AcNPV vector, a human collagen cDNA can be incorporated into a silkworm genomic DNA by homologous recombination through constructing a targeting vector by incorporating a human collagen cDNA into an AcNPV genomic DNA with a conventional method and infecting a larval silkworm with this constructed vector. The site into which the human collagen cDNA is incorporated by homologous recombination may be for example, within a silk protein gene which may include the fibroin H chain, the fibroin L chain, P25 and sericin, or may be an arbitrary genomic DNA region other than the silk protein genes.

In instances where the incorporation site is within the silk protein gene, DNA sequences which are homologous to two sites of arbitrary regions within the silk protein gene are ligated respectively before and behind of a human collagen cDNA to construct an AcNPV targeting vector. By infection of this targeting vector, a human collagen cDNA is incorporated downstream of an endogenous expression regulatory sequence of a silkworm leading to expression of human collagen from the cDNA through the action of endogenous silk protein promoter/ enhancer. Further, in this case, a human collagen cDNA can be also incorporated into a silk protein gene so that a fusion protein of a human collagen with a silk protein can be synthesized. For example, a fusion protein of a human collagen with a fibroin L chain can be synthesized through incorporating a human collagen cDNA immediately before a termination codon in the seventh exon of the fibroin L chain gene, such that an amino acid frame is serially formed. Into the AcNPV vector in this instance, are inserted DNA sequences of approximately 0.5 kb to 6.0 kb respectively, which are homologous to genomic DNAs of upstream and downstream from the seventh exon of the fibroin L chain, before and behind of the collagen cDNA.

In addition, when a human collagen cDNA is subjected to homologous recombination to an arbitrary genomic DNA sequence other than the silk protein genes, a human collagen expression cassette is produced with a silk protein gene promoter ligated upstream of a human collagen cDNA. Then DNA sequences, which are homologous to approximately 0.5 kb to 6.0 kb respectively of upstream and downstream of the silkworm genomic DNA region to be subjected to homologous recombination of the expression cassette, are ligated before and behind of the expression cassette, and the ligated product is incorporated into the AcNPV vector to construct a targeting vector. In either case where the homologous recombination site is in the silk protein gene, or is in an arbitrary genomic sequence other than silk protein genes, it is also possible that a marker gene is simultaneously incorporated together with a human collagen cDNA, and selection of a transformed silkworm in the next generation (F1) and the generation after next (F2) is facilitated. Examples of the marker gene include for example, fluorescent protein genes such as GFP. Further, examples of the promoter to permit the expression of the marker gene include for example, silkworm actin promoter and *Drosophila* HSP70 promoter.

Other embodiment for generating a transformed silkworm having a human collagen cDNA incorporated therein concerns transformation in which DNA transposon derived from an insect is utilized. DNA transposon derived from an insect such as *piggyBac, mariner* (Insect Mol. Biol. 9: 145-155, 2000) or *Minos* (Insect Mol. Biol. 9: 277-281, 2000) is known to exhibit a transfer activity in silkworm cells, and thus transformation of a silkworm is enabled by a plasmid vector produced on the basis of such DNA transposon. In particular, transformation of a silkworm was successfully effected in fact by microinjection of a plasmid vector produced on the basis of *piggyBac* into a silkworm egg (Nat. Biotechnol. 18: 81-84, 2000). When DNA transposon derived from an insect is utilized, the foreign gene is incorporated into an arbitrary sequence in a silkworm genome. Therefore, for permitting the expression in a silk gland of the human collagen cDNA which was incorporated into a silkworm genomic sequence, to produce a human collagen expression cassette is required which was previously ligated upstream of the human collagen cDNA, with promoter and enhancer derived from silk protein genes which may include the fibroin H chain, the fibroin L chain, P25 and sericin.

Moreover, when a fusion polynucleotide encoding human collagen with a silk protein is incorporated, an expression cassette is produced with a promoter and enhancer derived from a silk protein gene ligated upstream of this fusion polynucleotide. Taking *piggyBac* as an example, characteristics and process for introducing a human collagen expression cassette are explained below, however, the DNA transposon derived from an insect which may be used in the invention is not limited to *piggyBac*, but other DNA transposon may be used which includes *mariner* and *Minos*. Process for introducing a human collagen expression cassette when such transposon other than *piggyBac* is used is essentially similar to the process as described below in which *piggyBac* is used.

The *piggyBac* is DNA transposon isolated from TN-368 which is a cultured cell derived from a Lepidoptera insect, *Trichoplusia ni*. It is composed of transposase ORF which is located in the middle part thereof and inverted terminal repeats of 13 bp which are positioned on both ends, and has the length of about 2.5 kb. A transposase protein is synthesized from the transposase ORF. By the action of a transposase, the region sandwiched between the inverted terminal repeats (*piggyBac* itself) is transferred to a targeted sequence, TTAA (Virology 161: 8-17, 1989). For insertion of a human collagen expression cassette into a silkworm genomic sequence utilizing such property of *piggyBac*, similar process to the process of for example, Tamura et al. (Nat. Biotechnol. 18: 81-84, 2000), may be carried out. More specifically, a pair of the inverted terminal repeats of *piggyBac* is incorporated into an appropriate plasmid vector, thereby inserting a human collagen expression cassette such that it is sandwiched between the pair of the inverted terminal repeats. Then this plasmid vector is microinjected into a silkworm egg together with a transposase expression vector (a helper plasmid) of *piggyBac*. This helper plasmid is a recombinant plasmid vector with deletion of one or both of the inverted terminal repeats of *piggyBac*, and substantially having transposase gene region of *piggyBac* alone incorporated therein. In this helper plasmid, a promoter which may be utilized for allowing expression of transposase may be an authentic transposon promoter as it is, or may be a silkworm actin promoter or *Drosophila* HSP70 promoter or the like. In order to facilitate the screening of silkworms in the next generation, a marker gene may be concomitantly incorporated into the vector having the collagen expression cassette incorporated. In this instance, a promoter sequence such as e.g., a silkworm actin promoter, *Drosophila* HSP70 promoter or the like is incorporated upstream of the marker gene, and the expression of the marker gene is activated on behalf of inserted promoter.

Selection of a transformed silkworm from silkworms in the F1 generation, and from silkworms in additional F2 generation in case where the AcNPV vector is used, may be performed using for example, a PCR method or a Southern blot method. In addition, when a marker gene was incorporated, selection using its phenotypic character is also possible. For example, when a fluorescent protein gene such as GFP was utilized as a marker gene, an excitation light is irradiated on eggs or larvae of silkworms in the F1 or F2 generation, and selection can be perfected by detecting fluorescence emitted from the fluorescent protein. The silkworm selected in such a manner is a transformed silkworm having the human collagen cDNA incorporated in its chromosome. Therefore, the human collagen cDNA is transmitted without disappearance also in offspring generated by mating of these silkworms with wild type silkworms, or of transformed silkworms with each other. Thus, production of human collagen or a fusion protein of collagen with a silk protein is permitted throughout the generations.

When a polynucleotide encoding prolyl hydroxylase is introduced to the transformed silkworm which produces human collagen as described above in a mode to enable the expression in a silk gland, completely heat stable human collagen can be produced at a physiological temperature. The prolyl hydroxylase polynucleotide to be introduced (e.g., cDNA) may be a polynucleotide derived from a human or any other animal, or may be a prolyl hydroxylase polynucleotide derived from a silkworm. The prolyl hydroxylase is a complex of the α subunit and the β subunit. Unless this complex is formed, no enzymatic activity is caused. The α subunit is a subunit having an enzymatic activity, and originally exists in a cell which produces collagen. To the contrary, the β subunit is a polypeptide which is identical to protein disulfide isomerase which is an enzyme that catalyzes structural conversion of a disulfide bond of a protein, and universally exists in all cells in a relatively large amount Since a silkworm silk gland cell does not produce a large amount of collagen, only a small amount of the α subunit is present, but a relatively large amount of β subunit is present therein. For introducing a prolyl hydroxylase polynucleotide to a silkworm and allowing expression of prolyl hydroxylase in a silkworm silk gland, a process in which a prolyl hydroxylase polynucleotide derived from an animal such as a human as described above is used, or a process in which a prolyl hydroxylase polynucleotide of a silkworm is used may be employed. When a prolyl hydroxylase polynucleotide derived from an animal such as a human is used, two kinds of polynucleotides respectively encoding the α subunit and the β subunit are required. Because the β subunit of an insect scarcely forms an active complex with the α subunit derived from an animal such as a human, an active enzyme can not be synthesized with merely expression of the α subunit alone derived from an animal such as a human. To the contrary, when a prolyl hydroxylase polynucleotide of a silkworm is used, use of the α subunit alone may be acceptable, because the α subunit which is expressed in a silk gland can form an active complex with endogenous β subunit that exists in a comparatively large amount.

This application provides a novel polynucleotide encoding a silkworm prolyl hydroxylase α subunit (SEQ ID NO: 2). This polynucleotide is a cDNA having a base sequence set out in SEQ ID NO: 1, and a DNA fragment isolated and purified from a genomic DNA and an RNA fragment. A genomic DNA fragment or an RNA fragment can be obtained by screening or PCR in which an oligonucleotide or the like is used which is produced on the basis of the base sequence set out in SEQ ID NO: 1.

In order to allow the expression of prolyl hydroxylase in a silkworm silk gland through introducing a polynucleotide encoding prolyl hydroxylase into a silkworm chromosome, a promoter capable of allowing gene expression in the silk gland is utilized. Examples of the promoter which satisfy this requirement include promoters of silk proteins that may include fibroin, P25 and sericin which are capable of allowing expression of a gene in only a silk gland, and promoters such as silkworm actin, *Drosophila* HSP70 and 1E1 of AcNPV which allow expression in any tissue. For introducing a prolyl hydroxylase polynucleotide into a silkworm chromosome, similar methods as one for introducing a human collagen cDNA as described above may be carried out, i.e., a gene targeting method in which AcNPV is used, or a method in which a plasmid vector produced on the basis of DNA transposon such as *piggyBac* is microinjected into a silkworm egg. For generating a silkworm having both polynucleotides of human collagen and prolyl hydroxylase, a prolyl hydroxylase polynucleotide may be introduced into a transformed silkworm having a human collagen polynucleotide, or on the contrary, a human collagen polynucleotide may be introduced into a transformed silkworm having a prolyl hydroxylase polynucleotide. Alternatively, a transformed silkworm having a human collagen polynucleotide alone, and a transformed silkworm having a prolyl hydroxylase polynucleotide are separately generated followed by mating therebetween, and then any transformed silkworm having both polynucleotides may be selected. Both polynucleotides can be introduced respectively with an AcNPV vector, or both polynucleotides can be introduced respectively with a DNA transposon vector, however, a human collagen polynucleotide may be introduced using an AcNPV targeting vector whilst a prolyl hydroxylase polynucleotide may be introduced using a DNA transposon plasmid vector. Alternatively, the reverse fashion thereof may be permissible.

The transformed silkworm having a human collagen cDNA synthesizes human collagen together with endogenous silk proteins when it reaches to the fifth-instar stage, and secretes human collagen in its cocoon as a part of the silk thread. Human collagen in the cocoon can be readily extracted with for example, 0.5 M acetic acid or the like. In addition, when human collagen was synthesized as a fusion protein with a silkworm fibroin L chain, extraction can be executed by putting it into a reducing state to cleave a disulfide bond between the fusion protein and a fibroin H chain. Further, when human collagen included in the cocoon is fibrous collagen and purification of only the triple helical region (atelocollagen) is intended, proteins in the cocoon are treated with a proteolytic enzyme such as pepsin. According to this manipulation, atelocollagen which is not digested with a proteolytic enzyme can be extracted, and many other proteins are subjected to digestion with pepsin. Therefore, the following purification can be readily performed. Moreover, human collagen can be also extracted and purified from the silk gland of the transformed silkworm in a similar manner to the instance of the cocoon. Because the silk gland can be easily separated by dissection of the silkworm, and almost of the proteins included therein are silk proteins, human collagen can be readily extracted and purified similarly to the case of the cocoon.

### Examples

The present invention is more specifically explained with reference to Examples relating to processes for producing human type III collagen, however, the present invention should not be construed as being limited to these Examples.

### Example 1

### Production of Transformed Silkworm by Gene Targeting Method Using AcNPV Vector

As cDNA encoding human type III procollagen, cDNA clone which previously obtained by the inventors of this application (JP-A-8-23979; GeneBank database Accession No. X14420) was used. A silkworm fibroin L chain gene and a silkworm genomic DNA sequence downstream of the same were isolated by the following method using base sequence of known silkworm fibroin L chain gene (Gene 100: 151-158, 1992; GeneBank database Accession No. M76430).

In the following description, base numbers in a human type III procollagen cDNA and a silkworm fibroin L chain gene are in accordance with the base numbers described in the aforementioned GeneBank database.

### (1) Isolation of Silkworm Fibroin L Chain Gene and Genomic DNA Sequence in Downstream Region of the Gene

Amplification of a gene fragment in the intron 6 of the fibroin L chain was performed using a PCR method. Oligonucleotides synthesized on the basis of the sequence of the aforementioned database (SEQ ID NO: 3 and SEQ ID NO: 4) were used as PCR primers, and a genomic DNA of a silkworm tokai x asahi strain was used as a template. Next, screening for a silkworm kinshu x showa strain genomic library which was constructed by λEMBL3 was performed using thus resulting DNA fragment as a probe according to a conventional method. As a consequence, a silkworm genomic DNA fragment (pRI/10k) was obtained which contains a region spanning about 15 kb downstream from the base number 9600 of the fibroin L chain gene.

### (2) Production of Restriction Enzyme Recognition Sequence by Site-Directed Mutagenesis

In order to ligate the type III procollagen cDNA into the exon 7 of a fibroin L chain gene, a new restriction enzyme site was provided to a fibroin gene by site-directed mutagenesis. For the mutagenesis, Mutagenesis Kit of Clontech was used. A primer for the mutagenesis (SEQ ID NO: 5) was designed such that a restriction enzyme XhoI site (positions 8-12 in SEQ ID NO: 5) is provided immediately before the stop codon of the fibroin L chain gene exon 7. As a template plasmid, a plasmid obtained by subcloning of an EcoRI-SphI fragment from pRI/10k (base number: 12000-14800) into pUC18 (pRISphI/2.9k) was used (Mut pRISphI-XhoI). In a similar manner, an XbaI site was newly provided by mutagenesis for inserting a fibroin L chain gene downstream fragment (base number: 14200-18900). A primer for the mutagenesis (SEQ ID NO: 6) was designed such that an XbaI site (positions 12-17 in SEQ ID NO: 6) is provided immediately before the stop codon of the fibroin L chain gene exon 7. As a template plasmid, pRISphI/2.9k which is similar to one described above was used (Mut pRISphI-XhoI).

A restriction enzyme XhoI site was introduced also into a base sequence encoding an aminopropeptide of a type III procollagen cDNA using a similar site-directed mutagenesis method as described above. Base sequence of the primer for the mutagenesis (SEQ ID NO: 7) corresponded to the base number of from 292 to 324 of the type III procollagen cDNA, which has the restriction enzyme XhoI site inserted therein (positions 17-22 in SEQ ID NO: 7).

### (3) Construction of Transfer Vector

A baculovirus transfer vector pBacPAK9 of Clontech was digested with restriction enzymes SmaI and EcoRI, and after ligation with a DNA fragment (EcoRV-EcoRI) containing *Drosophila* HSP70 promoter excised from pCaSepR-hsp (GeneBank Accession No. U59056) and an EGFP cDNA (Clontech) ligated thereto, the product was transformed to Escherichia coli DH5 α (pBacPAKhsEGFP). Ligation was conducted using Ligation Kit Ver. 1 (TaKaRa), and transformation and the like were carried out according to the conventional method.

A fragment of the insert sequence (EcoRV-XhoI) was excised from previously completed mutant plasmid Mut pRISphI-XhoI, and was ligated to the Xhol site of type III procollagen cDNA which had been constructed by mutagenesis. Subsequently, an SV40 polyadenylation signal sequence which was amplified from pCEP4 (Invitrogen) by PCR was inserted downstream of the collagen cDNA (BglI site). Then, thus completed fibroin-collagen-polyadenylation signal sequence fragment (EcoRV-BglI) was ligated to EcoRV-BglI sites of pBacPAKhsEGFP. Next, from a part of the fibroin L chain gene intron 2 to a part of the exon 7 (base number: about 10000-13100) was inserted in the EcoRV site (pBacPAKhsEGFP-Fib1).

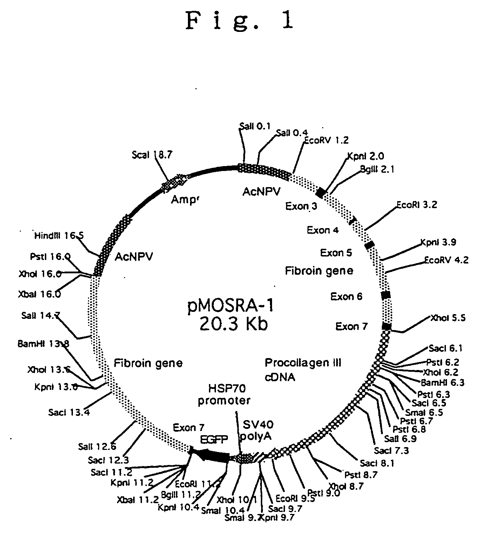

Subsequently, a fragment EcoRV-SphI of 1.7 kbp of the insert sequence (base number: about 13100-14800) was excised from the mutant plasmid Mut pRISphI-XbaI, and inserted to SmaI-SphI sites of pRI/10k (by partial digestion). An Xbal digestion fragment excised from thus completed MutRI/10k (a part of the fibroin gene exon 7 and downstream region thereof/ base number: about 14200-18900) was inserted to pBacPAKhsEGFP-Fib1 (pMOSRA-1: Fig. 1).

### (4) Production of Recombinant Virus

A recombinant virus was produced by cotransfection of thus produced targeting vector pMOSRA-1 and baculovirus DNA into insect cultured cell Sf9. After adding 4 µl of lipofectin (Gibco) and 4 µl of water to 7 µl of 0.4 µg/µl pMOSRA-1 and 1 µl of 0.1 µg/µl linear baculovirus DNA (Pharmingen) and mixing well, the mixture was dropped onto 1 × 10⁶ cells of Sf9 cell which had been replaced with a serum free medium SF900-II (Gibco), and cultured. After 24 hrs passed, 1 ml of a Grace's medium supplemented with 10% serum was added thereto followed by culture for additional 3 days. Then the culture supernatant was recovered.

### (5) Screening and Refinement of Recombinant Virus

To 1 × 10⁶ cells of Sf9 cell was added 100 µl of the aforementioned viral liquid to allow infection at 28°C for 1 hour. The supernatant was removed, and thereto was added 1 ml of a Grace's medium containing 1% low melting agarose solution (SEAPLAQUE, FMC) to permit hardening. After culture for 3 days, an excitation light having the wavelength of 360 nm was irradiated on the resulting plaques. Ten plaques generating green fluorescence, i.e., plaques of cells which express EGFP were excised on the block with the agar. Next, recombinant viruses recovered from these plaques were again infected to 1 × 10⁶ cells of Sf9 cell, and were subjected to extraction of intracellular DNA, followed by dot blotting of the extracted DNA. As the probe, two kinds of probes, i.e., a probe which recognizes the fibroin L chain gene and a probe which recognizes a procollagen cDNA were employed. Consequently, viral clones derived from 4 plaques were revealed to be positive. Then these positive viruses were infected to 1 × 10⁶ cells of Sf9 cell, and the culture supernatant was recovered after three days passed. The viruses in the culture supernatant were again infected similarly to the Sf9 cells to obtain a viral stock having a high titer.

### (6) Infection of Recombinant Baculovirus to Larval Silkworm of the Fifth-instar Stage

A female larval silkworm on the first day of the fifth-instar stage was subcutaneously injected 50 µl of the viral liquid (5 × 10⁶ pfu). On the 3rd and 4th days after pupation of the inoculated larva, 10 µl of 20-hydroxyecdysone dissolved in methanol (2 mg/ml) was administered. After the eclosion, mating was conducted with a male normal silkworm moth and then oviposition was permitted.

### (7) Screening of F1 Silkworm

F1 eggs (100 to 300 eggs) laid by one female silkworm were classified into 1 group, and about 50 eggs from each group were sampled while the remaining eggs of respective groupsleft to be kept. DNA was extracted from the sampled eggs by a conventional method, and subjected to determination of whether or not any foreign gene was present by a PCR method. PCR was performed such that the SV40 polyadenylation signal was detected using a primer set (SEQ ID NO: 8 and 9), or such that the HSP70 promoter was detected using a primer set (SEQ ID NO: 10 and 11).

As a result of screening of eggs from 1800 groups in total, 15 groups were positive. Remaining eggs in the positive groups were hatched, and thus hatched F1 larvae were kept until the fifth-instar stage. On the third day of the fifth-instar stage, about 100 µl of a body fluid was collected from each larva. From these body fluids were separated body fluid cells by centrifugation. After extracting the DNA from these cells, screening by a PCR method was conducted. As a consequence of screening for total 3400 larvae of the fifth-instar stage, 8 larvae were positive. Keeping of these silkworms was continued, and the eclosed silkworm moth was mated with a normal silkworm moth to permit oviposition.

### (8) Screening of F2 Silkworm

Screening of F2 silkworms was carried out with green fluorescence. An excitation light having the wavelength of 360 nm was irradiated on the first-instar larvae, and selection was executed for individuals generating green fluorescence of EGFP, i.e., individuals who express EGFP which was inserted as a marker gene. As a result of screening of 8 groups of F2 eggs, two positive silkworms could be obtained.

### (9) Detection of Recombinant Human Collagen

Positive F2 silkworms were kept until the spinning stage, and the proteins in the cocoon were extracted by adding thereto an SDS-sample buffer (0.125 M Tris-HCl buffer, pH 6.8/ 4% SDS/ 10% 2-mercaptoethanol/ 20% glycerol) and mixing followed by a heat treatment at 100°C for 5 min. This sample was applied to SDS polyacrylamide gel electrophoresis (Nature 227: 680-685, 1970), and the electrophoresed proteins were transferred to a nitrocellulose membrane BA85 (S&S Corporation) according to the method of Matudaira et al. (J. Biol. Chem. 261: 10035-10038, 1987). Next, the nitrocellulose membrane with the transferred proteins was subjected to a treatment in a blocking solution (3% BSA/ 50 mM Tris-HCl buffer, pH 7.5/ 150 mM NaCl) at 4°C for 16 hrs. Thereafter, a reaction was allowed with a 200 x diluted anti-human/ bovine type III collagen antibody in the blocking solution at room temperature for 1 hour. Proteins which react with these antibodies were detected with VECTASTAIN ABC kit (Vector Laboratories Company). Consequently, human type III collagen was detected from the cocoon.

### Example 2

### Production of Transformed Silkworm by Microinjection of piggyBac Vector into Egg

### (1) Production of piggyBac Plasmid Vector

After digesting a human type III procollagen cDNA (base number: 92-4550; GeneBank database Accession No. X14420) with XhoI to remove the region corresponding to the base number of 1075-3545, a mini type III collagen cDNA was produced through ligating the truncated ends by self ligation. This mini collagen cDNA was constituted from base sequences coding for an aminopropeptide, a part of a triple helical region (about one fifth of the entire collagen triple helical region) and a carboxyl propeptide, and the following three kinds of vectors were constructed on the basis thereof.

### i) pMOSRA-4B

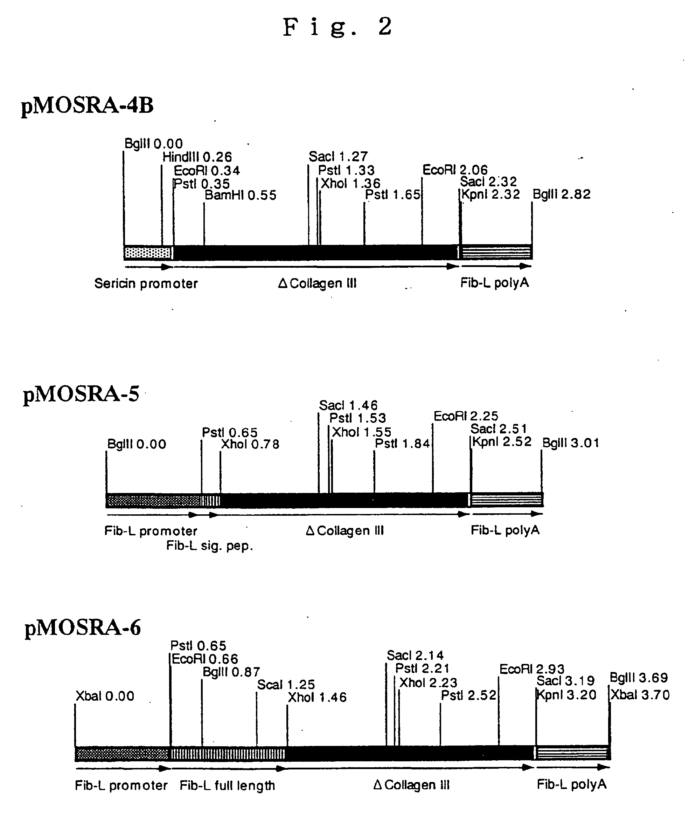

The insert DNA fragments included in this vector are constituted from a silkworm sericin prompter, a mini collagen cDNA, and a silkworm fibroin L chain polyadenylation signal. The silkworm sericin promoter (base number: 1299-1622; GeneBank database Accession No. AB007831) was isolated by PCR using a primer having an added BglII recognition sequence at its 5' end (SEQ ID NO: 12) and a primer (SEQ ID NO: 13) with a silkworm genomic DNA as a template. The isolated promoter was ligated upstream of the mini collagen cDNA. Furthermore, a silkworm fibroin L chain polyadenylation signal (base number: 14141-14624; GeneBank database Accession No. M76430) was isolated by PCR using a primer (SEQ ID NO: 14) and a primer having an added BgIII recognition sequence at its 5' end (SEQ ID NO: 15) with a silkworm genomic DNA as a template. The isolated signal was ligated downstream of the mini collagen cDNA (Fig. 2). After digesting both ends of thus resulting insert DNA fragment with BgIII, protruding ends thereof was blunted by treatment with T4DNA polymerase. Thereafter, this blunt-ended fragment was inserted into a pPIGA3GFP vector, which had been digested with XhoI and then blunted (Nat. Biotechnol. 18: 81-84, 2000). An EGFP cDNA as a marker and a silkworm actin (A3) promoter for allowing the expression of this cDNA are incorporated in the pPIGA3GFP vector.

### ii) pMOSRA-5

The insert DNA fragments included in this vector are constituted from a silkworm fibroin L chain promoter, a silkworm fibroin L chain signal peptide cDNA, a mini collagen cDNA, and a silkworm fibroin L chain polyadenylation signal. The silkworm fibroin L chain signal peptide cDNA (base number: 28-160; GeneBank database Accession No. X17291) was isolated by PCR using a primer (SEQ ID NO: 16) and a primer having an incorporated XhoI recognition sequence at its 5' end (SEQ ID NO: 17) with a cDNA derived from a silkworm silk gland as a template. In addition, in a similar manner to that described in Example 1 (2), an Xhol recognition sequence was introduced into encoding region of procollagen aminopropeptide of a mini collagen cDNA, and the region from the 5' end to XhoI cleavage site of this modified cDNA was substituted for isolated fibroin L chain signal peptide cDNA. Furthermore, the silkworm fibroin L chain promoter (base number: 428-1061; GeneBank database Accession No. M76430) was isolated by PCR using a primer having an added XbaI and BgIII recognition sequences at its 5' end (SEQ ID NO: 18) and a primer (SEQ ID NO: 19) with a silkworm genomic DNA as a template. Thereafter the isolated promoter was ligated upstream of the silkworm fibroin L chain signal peptide cDNA, and the silkworm fibroin L chain polyadenylation signal was ligated downstream of the mini collagen cDNA (Fig. 2). Both ends of thus resulting insert DNA fragment were digested with BglII, and the protruding ends were blunted by T4 DNA polymerase. Thereafter, it was inserted into the pPIGA3GFP vector, which had been digested with XhoI and then blunt ended.

### iii) pMOSRA-6

The insert DNA fragments included in this vector are constituted from a silkworm fibroin L chain promoter, a silkworm fibroin L chain full length cDNA, a mini collagen cDNA, and a silkworm fibroin L chain polyadenylation signal. The cDNA covering full length of silkworm fibroin L chain (base number: 30-820; GeneBank database Accession No. X17291) was isolated by PCR using a primer (SEQ ID NO: 20) and a primer having an incorporated XhoI recognition sequence at its 5' end (SEQ ID NO: 21) with a cDNA derived from a silkworm silk gland as a template. Next, in a similar method to that of producing pMOSRA-5, the aminopropeptide-encoding region from 5' end to the modified XhoI cleavage site in mini collagen cDNA was substituted for the isolated cDNA. In addition, the silkworm fibroin L chain promoter was ligated upstream of the silkworm fibroin L chain cDNA, and the silkworm fibroin L chain polyadenylation signal was ligated downstream of the mini collagen cDNA (Fig. 2). Both ends of thus resulting insert DNA fragment were digested with XbaI, and the protruding ends were additionally blunted with T4 DNA polymerase. Thereafter, it was inserted into the pPIGA3GFP vector, which had been digested by XhoI and then blunted.

### (2) Microinjection of Plasmid Vector into Silkworm Egg

After purifying the aforementioned three kinds of mini collagen vectors (pMOSRA-4B, pMOSRA-5, pMOSRA-6) by a cesium chloride ultracentrifuge method, these three kinds of vectors and pHA3PIG which is a helper plasmid (Nat. Biotechnol. 18: 81-84, 2000) were admixed. After being subjected to ethanol precipitation, the mixture was dissolved in an injection buffer (0.5 mM phosphate buffer, pH 7.0, 5 mM KCl) such that each concentration of the mini collagen vector became 66.7 µg/ml (200 µg/ml in total) and the concentration of pHA3PIG became 200 µg/ml. This DNA solution was microinjected into silkworm eggs in a preblastoderm stage of 2 to 8 hrs post oviposition (silkworm embryos), in a fluid volume of about 15 to 20 nl per one egg. Microinjection was carried out to 1078 eggs in total.

### (3) Screening of F1 Larvae

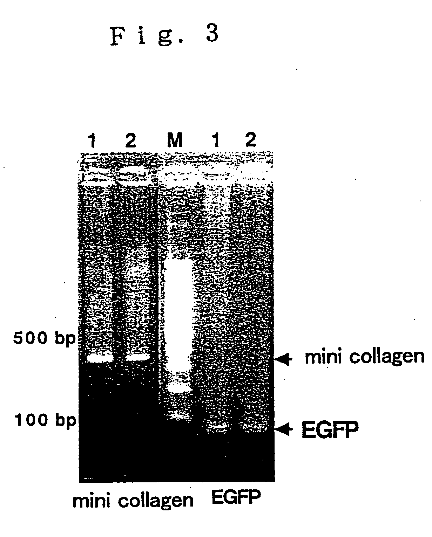

When eggs having the microinjected vector DNA were incubated at 25°C, 518 eggs were hatched. The hatched eggs were subsequently kept, and 413 reproductive adults were obtained, which were mated to give 213 groups of F1 egg batches. Next, these F1 eggs were hatched, and the hatched larvae were observed by a fluorescence microscope for each group. As a consequence, larvae generating green fluorescence were obtained from 26 groups. Number of positive silkworms included in the positive groups was 1 to 30 per one group, and summation thereof was 240. These positive F1 silkworms were kept and mated with a wild type silkworm to obtain silkworms of the additional next generation (F2). Among the F2 silkworms, it was ascertained that about half silkworms generated green fluorescence, and that the incorporated EFGP and mini collagen genes were transmitted to the next generation without drop out, according to Mendelian rule. Moreover, when a genomic DNA was extracted from F1 silkworm adults after the oviposition followed by amplification of a mini collagen DNA fragment by PCR using a primer set (SEQ ID NOs: 22 and 23) and of an EGFP fragment by PCR using a primer set (SEQ ID NOs: 24 and 25), the mini collagen and EGFP DNA fragments could be detected from the positive silkworms which generate green fluorescence (Fig. 3). Additionally, Southern blot analysis was performed to confirm that these exogenous genes were incorporated into the silkworm genomic DNA.

### (4) Detection of Mini Collagen mRNA and Recombinant Mini Collagen

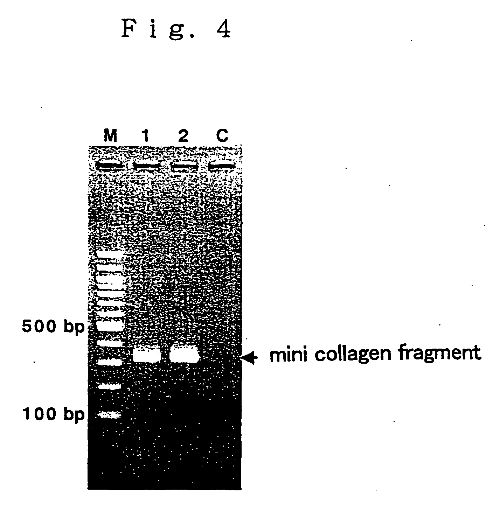

A positive F1 larva reached to the fifth-instar stage was submitted to dissection to remove a silk gland, and total RNA was extracted by an acid guanidine phenol chloroform method. PCR was conducted using a cDNA as a template, which was obtained by reverse-transcription of the RNA in an amount of 200 ng from the extracted total RNA, with 30 cycles under the reaction condition of: 94°C for 1 minute, 60°C for 1 minute and 72°C for 1 minute (RT-PCR). As a primer, the aforementioned primer set (SEQ ID NOs: 22 and 23) was used. Consequently, the mini collagen mRNA could be detected, and it was ascertained that the mini collagen gene incorporated into the silkworm genomic DNA was expressed in silk gland cells (Fig. 4).

Next, detection of the recombinant mini collagen protein was attempted. Positive F1 silkworms were kept until the spinning stage, and the proteins in the discharged cocoon were extracted by adding thereto an SDS-sample buffer (0.125 M Tris-HCl buffer, pH 6.8/ 4% SDS/ 10% 2-mercaptoethanol/ 20% glycerol) and mixing followed by a heat treatment at 100°C for 5 min. This extracted sample was applied to SDS polyacrylamide gel electrophoresis (Nature 227: 680-685, 1970), and the electrophoresed proteins were transferred to a nitrocellulose membrane BA85 (S&S Corporation) according to the method of Matudaira et al. (J. Biol. Chem. 261: 10035-10038, 1987). Next, the nitrocellulose membrane with the transferred proteins was subjected to a treatment in a blocking solution (3% BSA/ 50 mM Tris-HCl buffer, pH 7.5/ 150 mM NaCl) at 4°C for 16 hrs. Thereafter, a reaction was allowed with a 200 x diluted anti-human/ bovine type III collagen antibody in the blocking solution at room temperature for 1 hour. Proteins which react with these antibodies were detected with VECTASTAIN ABC kit (Vector Laboratories Company). Consequently, the recombinant mini collagen protein could be detected from the cocoon.

### Example 3

### Cloning of Silkworm Prolyl hydroxylase α Subunit cDNA

First, a partial sequence of silkworm prolyl hydroxylase α subunit cDNA was cloned by a degenerate PCR method using mixed primers. By comparison to amino acid sequences deduced from each reported prolyl hydroxylase α subunit gene of human, fruit fly and nematode, mixed primers P3 (SEQ ID NO: 26) and P5II (SEQ ID NO: 27) were designed having base sequences which can be degenerated from the amino acid sequence conserved among them. In the mixed primer sequences, n indicates a, c, g or t; r indicates a or g; y indicates c or t; s indicates c or g; and w indicates a or t. Subsequently, for the purpose of preparing a template for PCR, total RNAs were extracted respectively from BmN4 cells which are silkworm culture cells and larval silkworm of the second-instar stage. PCR was conducted using a cDNA as a template, which was obtained by reverse-transcription of the RNA in an amount of 200 ng from the extracted total RNA, with 40 cycles under the reaction condition of: 94°C for 1 minute, 58°C for 1 minute and 72°C for 1 minute. As a consequence, an amplification product of about 150 bp was confirmed on electrophoresis for both of the BmN4 cells and larval silkworm of the second-instar stage. The amplification product was subcloned into pCR2.1 (Invitrogen), and the base sequence was determined by a dideoxy method. Thus, it was revealed that this cDNA fragment was a partial fragment of the silkworm prolyl hydroxylase cDNA because it had high homology with prolyl hydroxylase α subunits of other animals in both respect of the base sequence level and the amino acid sequence level predicted therefrom.

Then, for the purpose of cloning the full length cDNA, upstream and downstream of thus resulting silkworm prolyl hydroxylase α subunit cDNA fragment were isolated by a RACE (Rapid Amplification cDNA Ends) method. Primer GSP1 for 5' RACE (SEQ ID NO: 28) and primer GSP2 for 3' RACE (SEQ ID NO: 29) were designed on the basis of the base sequence of the cDNA fragment. RACE was carried out using SMART™ RACE cDNA Amplification Kit of Clontech. As a consequence of the RACE, cDNA fragments of about 1.7 kb in the 5' region and of about 1.2 kb in the 3' region could be ascertained on electrophoresis. These cDNA fragments were subcloned similarly as described above, and the base sequences were analyzed. Thus, the cDNA fragments contained the entire sequence encoding the silkworm prolyl hydroxylase α subunit. The base sequence of the resulting full length cDNA is set out in SEQ ID NO: 1, and the amino acid sequence of the silkworm prolyl hydroxylase α subunit encoded by this cDNA is set out in SEQ ID NO: 2.

### Industrial Applicability

As explained in detail hereinabove, according to the present invention, a transformed silkworm which produces recombinant human collagen as a part of proteins included in the cocoon or the silk gland; and recombinant human collagen produced by this silkworm are provided. Since recombinant human collagen is collected from the cocoon discharged by the transformed silkworm or from the silk gland of the transformed silkworm, extraction thereof is easy, and hence, collagen having high purity can be readily obtained. In addition, because the recombinant human collagen produced by the transformed silkworm is safe collagen which is free from any fear of the contamination with pathogens such as viruses or prions and exhibits no antigenicity toward humans. Thus, it can be utilized in various industrial fields including medicines, foods, cosmetics and the like.

## Claims

1. A transformed silkworm, which comprises a polynucleotide encoding human collagen within the genomic DNA and produces recombinant human collagen as a part of proteins in the cocoon or the silk gland.

2. The transformed silkworm of claim 1 further comprising a polynucleotide encoding at least one of the α subunit and the β subunit of prolyl hydroxylase within the genomic DNA.

3. The transformed silkworm of claim 2, wherein the polynucleotide encoding the prolyl hydroxylase α subunit is at least a coding region of a DNA fragment having a base sequence of SEQ ID NO: 1.

4. A transformed silkworm, which comprises a polynucleotide encoding a fusion protein of human collagen with a silkworm silk protein within the genomic DNA and produces said fusion protein as a part of proteins in the cocoon or the silk gland.

5. The transformed silkworm of claim 4 further comprising a polynucleotide encoding at least one of the α subunit and the β subunit of prolyl hydroxylase within the genomic DNA.

6. The transformed silkworm of claim 5, wherein the polynucleotide encoding the prolyl hydroxylase α subunit is at least a coding region of a DNA fragment having a base sequence of SEQ ID NO: 1.

7. A process for producing recombinant human collagen, which comprises isolating and purifying recombinant human collagen from the cocoon or the silk gland of the transformed silkworm of claim 1, 2 or 3.

8. A process for producing recombinant human collagen, which comprises isolating a fusion protein of recombinant human collagen with a silkworm silk protein from the cocoon or the silk gland of the transformed silkworm of claim 4, 5 or 6, and isolating and purifying recombinant human collagen from the fusion protein.

9. A fusion protein of recombinant human collagen with a silkworm silk protein produced by the transformed silkworm of claim 4, 5 or 6.

10. A targeting vector derived from *Autographa californica* nuclear polyhedrosis virus, which comprises a polynucleotide encoding human collagen .

11. The targeting vector of claim 10, wherein the polynucleotide is a human collagen expression cassette.

12. The targeting vector of claim 11, wherein the human collagen expression cassette is a fusion polynucleotide including a polynucleotide encoding human collagen ligated under the control of an expression regulatory sequence of a gene for silkworm silk protein.

13. The targeting vector of claim 11, wherein the human collagen expression cassette is a fusion polynucleotide including a polynucleotide encoding a silkworm silk protein and a polynucleotide encoding human collagen ligated under the control of an expression regulatory sequence of a gene for silkworm silk protein.

14. A targeting vector derived from *Autographa californica* nuclear polyhedrosis virus, which comprises a polynucleotide encoding at least one of the α subunit and the β subunit of prolyl hydroxylase.

15. The targeting vector of claim 14, wherein the polynucleotide encoding the prolyl hydroxylase α subunit is at least a coding region of a DNA fragment having a base sequence of SEQ ID NO: 1.

16. A recombinant plasmid vector having a human collagen expression cassette in a region sandwiched between a pair of inverted terminal repeats of DNA transposon derived from an insect.

17. The recombinant plasmid vector of claim 16, wherein the human collagen expression cassette is a fusion polynucleotide including a polynucleotide encoding human collagen ligated under the control of an expression regulatory sequence of a gene for silkworm silk protein.

18. The recombinant plasmid vector of claim 16, wherein the human collagen expression cassette is a fusion polynucleotide including a polynucleotide encoding a silkworm silk protein and a polynucleotide encoding human collagen ligated under the control of an expression regulatory sequence of a gene for silkworm silk protein.

19. A recombinant plasmid vector having a polynucleotide encoding at least one of the α subunit and the β subunit of prolyl hydroxylase in a region sandwiched between a pair of inverted terminal repeats of DNA transposon derived from an insect.

20. The recombinant plasmid vector of claim 19, wherein the polynucleotide encoding the prolyl hydroxylase α subunit is at least a coding region of a DNA fragment having a base sequence of SEQ ID NO: 1.

21. A set of vectors, which comprises a recombinant plasmid vector of claim 16, 17 or 18, and a recombinant plasmid vector having a polynucleotide encoding transposase of the transposon.

22. A set pf vectors, which comprises a recombinant plasmid vector of claim 19 or 20, and a recombinant plasmid vector having a polynucleotide encoding transposase of the transposon.

23. A polynucleotide encoding the α subunit of silkworm prolyl hydroxylase, which has the amino acid sequence of SEQ ID NO: 2.

24. The polynucleotide of claim 23, which is a DNA fragment having a base sequence of SEQ ID NO: 1.
